# EUROPÄISCHE PATENTSCHRIFT

(11) **EP 4 195 336 B1**
(45) Veröffentlichungstag und Bekanntmachung des Hinweises auf die Patenterteilung: **22.04.2026**
(21) Anmeldenummer: 22207211.8
(22) Anmeldetag: 14.11.2022
(51) Int. Cl.: H01M 8/04089, G01N 27/00, G01N 33/00

(54) **ELEKTRISCHES GERÄT FÜR DEN EINSATZ IN EINEM WASSERSTOFF-SYSTEM**
ELECTRICAL APPARATUS FOR USE IN A HYDROGEN SYSTEM
APPAREIL ÉLECTRIQUE POUR UTILISATION DANS UN SYSTÈME D'HYDROGÈNE

(30) Priorität: 09.12.2021 DE 102021214054
(43) Veröffentlichungstag der Anmeldung: 14.06.2023
(73) Patentinhaber: Robert Bosch GmbH, 70442 Stuttgart (DE)
(72) Erfinder: Brauer, Ingo, 71665 Vaihingen (DE); Hueftle, Gerhard, 71546 Aspach (DE)

(56) Entgegenhaltungen:
- EP-A1- 1 921 445
- CN-U- 208 937 567
- DE-A1- 102009 030 180
- DE-A1- 102011 008 720
- DE-T5- 112005 000 251
- KR-A- 20070 070 346
- SU-A1- 1 247 736

## Beschreibung

### Stand der Technik

Mit Wasserstoff betriebene Systeme, beispielsweise Brennstoffzellensysteme oder Systeme mit Wasserstoffverbrennung, verwenden elektrisch betriebene Geräte, die in Kontakt mit einem Wasserstoff enthaltenden Betriebsgas eingesetzt werden. Handelt es ich bei dem Betriebsgas beispielsweise um das Anodengas eines Brennstoffzellensystems, so kann der Wasserstoffgehalt im Bereich von 50 bis 100 Volumenprozent Wasserstoff liegen. Die elektrischen Geräte können Betriebskomponenten aufweisen, die mittels einer elektronischen Steuerschaltung angesteuert werden. Derartige Betriebskomponenten können beispielsweise in Kontakt mit dem Betriebsgas stehende Sensorelemente von Sensoren oder elektrisch betriebene Aktuatoren sein, die ebenfalls Baugruppen enthalten können, die mit dem Betriebsgas in Kontakt stehen. Die elektronische Steuerschaltung des Gerätes ist in der Regel auf einem elektronischen Schaltungsteil in einem Gehäuseinnenraum des Gerätes verbaut und elektrisch mit dem Sensorelement bzw. mit Teilen des Aktuators verbunden.

Aus der DE 10 2014 212 430 A1 ist ein als Wasserstoff-Konzentrationssensor ausgebildetes elektrisches Gerät für den Einsatz in einem Wasserstoff-System bekannt, das ein Gehäuse mit einem an dem Gehäuse ausgebildeten Eintrittskanal für das Betriebsgas, wenigstens einen Gehäuseinnenraum, in dem ein elektronisches Schaltungsteil angeordnet ist, und eine mit dem elektronischen Schaltungsteil elektrisch verbundene, in dem Gehäuse angeordnete Betriebskomponente aufweist, wobei der Eintrittskanal über ein in dem Gehäuse angeordnetes erstes Dichtelement an die Betriebskomponente angebunden ist und die Betriebskomponente über den Eintrittskanal mit Betriebsgas beaufschlagbar ist, wobei das erste Dichtelement den Eintrittskanal gegenüber dem Gehäuseinnenraum abdichtet. Weitere Sensoren zur Bestimmung der Wasserstoffkonzentration sind beispielsweise aus der DE 10 2005 058 832 A1, der DE 10 2011 008 720 A1 oder der DE 10 2014 202 169 A1 bekannt. Derartige Sensorelemente zur Erfassung von Wasserstoff können beispielsweise in Wasserstoff-Brennstoffzellenfahrzeugen zum Einsatz kommen, um Wasserstoff zu detektieren, der aufgrund einer Beschädigung oder eines Defektes austritt, und um Warnsignale oder Schutzmaßnahmen auslösen zu können.

Darüber hinaus ist es auch bekannt, elektrisch betriebene Aktuatoren in Brennstoffzellensystemen einzusetzen. Hierbei kann es sich beispielsweise um elektrisch angesteuerte Ventile handeln, welche Strömungspfade innerhalb des Brennstoffzellensystems öffnen oder verschließen.

Ein Luft-Wasserstoff-Gemisch kann bei einem Wasserstoffanteil von etwa 4 % zündungsfähig werden. Ein unmittelbarer Kontakt zwischen dem Betriebsgas und der dem elektronischen Schaltungsteil zur Ansteuerung der Betriebskomponente ist daher unerwünscht, um eine Zündung eines möglicherweise zündfähigen Gasgemisches im Gehäuseinnenraum zu verhindern.

### Offenbarung der Erfindung

Die Erfindung stellt ein elektrisches Gerät für den Einsatz in einem System vor, welches mit einem Wasserstoff enthaltenden Betriebsgas betrieben wird. Das Gerät umfasst ein Gehäuse mit einem an dem Gehäuse ausgebildeten Eintrittskanal für das Betriebsgas und mit wenigstens einem Gehäuseinnenraum, in dem ein elektronisches Schaltungsteil angeordnet ist. Mit dem elektronischen Schaltungsteil ist eine in dem Gehäuse angeordneten Betriebskomponente elektrisch verbunden. Der Eintrittskanal ist über ein in dem Gehäuse angeordnetes erstes Dichtelement an die Betriebskomponente angebunden, so dass die Betriebskomponente über den Eintrittskanal mit Betriebsgas beaufschlagbar ist. Das erste Dichtelement dichtet den Eintrittskanal gegenüber dem Gehäuseinnenraum bis auf eine verbleibende Wasserstoffpermeabilität ab. Die Erfindung schlägt nun vor, dass das Gehäuse weiterhin wenigstens ein den Gehäuseinnenraum zum Außenraum hin abdichtendes zweites Dichtelement aufweist, wobei das zweite Dichtelement eine Wasserstoffpermeabilität aufweist, die gleich oder größer als die Wasserstoffpermeabilität des ersten Dichtelementes ist.

Unter Permeation wird im Kontext der vorliegenden Anmeldung ein Vorgang verstanden, bei dem ein gelöster Stoff einen Festkörper durchdringt oder durchwandert. Der Stoff durchdringt dabei den Festkörper durch Poren und molekulare Zwischenräume. Die Permeation besteht aus drei Teilschritten. Einer Sorption des Stoffes an der Grenzfläche des Festkörpers, einer Diffusion durch den Festkörper und einer Desorption auf der anderen Seite des Festkörpers. Antreibende Kraft ist ein Gradient des chemischen Potentials. Die Permeabilität wird als Maß der Permeation in der Einheit µg cm⁻² min⁻¹ angegeben.

Unter der Dicke eines Dichtelementes wird der Abstand zweiter Grenzflächen des Dichtelementes verstanden, wobei eine erste Grenzfläche einem Gas mit höherer Wasserstoffkonzentration und die zweite Grenzfläche auf einer von der ersten Grenzfläche abgewandten Seite des Dichtelementes angeordnet ist.

Unter einem Wasserstoff-System wird ein mit Wasserstoff betriebenes System, insbesondere ein Brennstoffzellensysteme oder ein System mit Wasserstoffverbrennung verstanden. Das Wasserstoff-System weist insbesondere wenigstens eine Strömungsleitung auf, in der ein Wasserstoff enthaltendes Betriebsgas strömt. Die Strömungsleitung kann beispielsweise im Anodenpfad einer Brennstoffzelle angeordnet sein.

### Vorteile der Erfindung

Um einen materiellen Kontakt des elektronischen Schaltungsteils mit dem Wasserstoff zu vermeiden, könnte zwar die Betriebskomponente beispielsweise mit einer für Wasserstoff undurchlässigen Kappe abgedeckt werden, jedoch erfordert dies einen sehr hohen konstruktiven Aufwand zur Implementierung der Kappe in den Gehäuseinnenraum, insbesondere auch zur Abdichtung der elektrischen Kontakte zwischen dem elektronischen Schaltungsteil und der Betriebskomponente, da diese Kontakte durch die Kappe hindurchgeführt werden müssten. Auch die Auswahl eines für Wasserstoff undurchlässigen Materials wie beispielsweise Stahl erhöht die Kosten.

Die Erfindung ermöglicht es vorteilhaft, den Gehäuseinnenraum des elektrischen Gerätes, in dem ein elektronisches Schaltungsteil angeordnet ist, mit einem einfachen ersten Dichtelement in preiswerter Weise gegenüber dem Eintrittskanal abzudichten, wobei das erste Dichtelement nicht hermetisch dicht sein muss. Als erstes Dichtelement kann beispielsweise ein Elastomer verwandt werden. Es kann sich beispielsweise um einen preiswert herstellbaren Dichtring oder einen Dichtkleber handeln. Obwohl ein Dichtelement aus einem Elastomer oder einem ähnlichen Material gasdicht ausgelegt werden kann, kann Wasserstoff das Material mittels Permeation mit der Zeit durchdringen. Daher kann Wasserstoff durch Permeation vom Eintrittskanal durch das erste Dichtelement hindurch in den Gehäuseinnenraum vordringen. Da das Gehäuse im Allgemeinen aus einem gasdichten Material (beispielsweise einem Thermoplast) besteht, welches gegenüber Wasserstoff eine geringere Permeabilität als das erste Dichtelement aufweist, könnte daher ohne Gegenmaßnahmen die Wassersstoffkonzentration im Gehäuseinnenraum langsam ansteigen. Dies ist jedoch nicht hinnehmbar, da dann im ungünstigsten Fall die elektronische Schaltung in dem Gehäuseinnenraum eine Zündung des zündfähigen Gemisches auslösen könnte.

Dadurch, dass das Gehäuse nun ein den Gehäuseinnenraum zum Außenraum hin abdichtendes zweites Dichtelement aufweist, wobei das zweite Dichtelement eine Wasserstoffpermeabilität aufweist, die gleich oder größer als die Wasserstoffpermeabilität des ersten Dichtelementes ist, wird vorteilhaft erreicht, dass Wasserstoff, der durch Permeation des ersten Dichtelementes in den Gehäuseinnenraum gelangt ist, wiederum durch Permeation des zweiten Dichtelementes aus dem Gehäuseinnenraum nach außen gelangt. Da die Wasserstoffpermeabilität des zweiten Dichtelementes gleich oder größer als die Wasserstoffpermeabilität des ersten Dichtelementes ist, gelangt daher mindestens so viel Wasserstoff durch das zweite Dichtelement aus dem Gehäuseinnenraum in den Außenraum, wie durch das erste Dichtelement in den Gehäuseinnenraum hineingelangt. Dadurch wird vorteilhaft vermieden, dass sich in dem Gehäuseinnenraum ein zündfähiges Gemisch bildet. Da die Permeabilität weiterhin insgesamt sehr klein ist, gelangt vorteilhaft nur sehr wenig Wasserstoff aus dem Gehäuse in den Außenraum und verteilt sich dort rasch, so dass sich außerhalb des Gehäuses kein zündfähiges Gemisch anreichern kann.

Vorteilhafte Ausführungsformen und Weiterbildungen der Erfindung werden durch die in den abhängigen Ansprüchen angegebenen Merkmale ermöglicht.

Vorteilhaft kann das zweite Dichtelement in eine Gehäusewand des Gehäuses eingelassen sein. Insbesondere kann das zweite Dichtelement als eine in die Gehäusewand eingelassene Membran ausgebildet sein. Die Membran kann eine Wasserstoffpermeabilität aufweist, die gleich oder größer als die Wasserstoffpermeabilität des ersten Dichtelementes ist.

Die Wasserstoffpermeabilität eines Dichtelementes wird durch sein Material, sowie die räumlichen Abmessungen definiert, wobei die räumlichen Abmessungen durch den Diffusionsquerschnitt und die Dicke gebildet werden. Ist das Dichtelement eine in die Gehäusewand eingelassene Membran, wo wird die Wasserstoffpermeabilität durch die Fläche der Öffnung in der Gehäusewand, in welche die Membran eingelassen ist, durch die Dicke der Membran und durch ihr Material bestimmt. Obwohl es grundsätzlich möglich ist, das zweite Dichtelement aus einem anderen Material als das erste Dichtelement auszubilden, kann das zweite Dichtelement jedoch auch insbesondere aus dem gleichen Material wie das erste Dichtelement bestehen. Eine gleiche oder höhere Wasserstoffpermeabilität des zweiten Dichtelementes kann dann in sehr einfacher Weise durch eine entsprechende Wahl der Größe der Membran eingestellt werden. Bei gleicher Dicke des ersten Dichtelementes und des zweiten Dichtelementes kann beispielsweise der Querschnitt der Membran, welcher einer Öffnung in der Gehäusewand abdichtet, in einfacher Weise entsprechend groß ausgebildet werden, um eine Wasserstoffpermeabilität zu erreichen, die gleich oder größer als die Wasserstoffpermeabilität des ersten Dichtelementes ist.

Besonders vorteilhaft kann das erste Dichtelement und/oder das zweite Dichtelement aus einem Elastomer-Dichtkörper bestehen. Das Elastomer dichtet gegen Gas und Feuchtigkeit in zuverlässiger Weise ab und ist preisgünstig einsetzbar. Das erste Dichtelement und/oder das zweite Dichtelement kann aber auch als eine Dichtklebeverbindung hergestellt sein. Auch der Dichtkleber kann ein Elastomer umfassen.

Das elektrische Gerät kann mit einem den Eintrittskanal umgebenden Stutzen an einen das Betriebsgas führenden Strömungskanal anschließbar sein. Die Montage des Gerätes erfolgt dann ein einfacher Weise durch Einführung des Stutzens in den Strömungskanal.

Besonders bevorzugt, kann die Betriebskomponente als ein Sensorelement zur Erfassung mindestens einer Eigenschaft des Betriebsgases ausgebildet sein.

Dabei kann das Sensorelement bevorzugt ausgewählt sein aus der folgenden Gruppe von Sensorelementen:
- ein Sensorelement zur Erfassung der Wasserstoffkonzentration von Wasserstoff in dem Betriebsgas,
- ein Sensorelement zur Erfassung des Drucks des Betriebsgases,
- ein Sensorelement zur Erfassung der Temperatur des Betriebsgases,
- ein Sensorelement zur Erfassung der Feuchtigkeit in dem Betriebsgas.

Darüber hinaus ist es aber auch möglich, als Betriebskomponente einen elektrisch betriebenen Aktuator zu verwenden. Bei dem Aktuator kann es sich beispielsweise um ein elektrisch bestätigtes Ventil und insbesondere ein Dosierventil handeln, welches für den Einlass oder Ablass von Gaskomponenten in dem Wasserstoff-System eingesetzt wird.

### Kurze Beschreibung der Zeichnungen

Nachfolgend werden mögliche Ausführungsformen der Erfindung unter Bezugnahme auf die beiliegende Zeichnung erläutert.

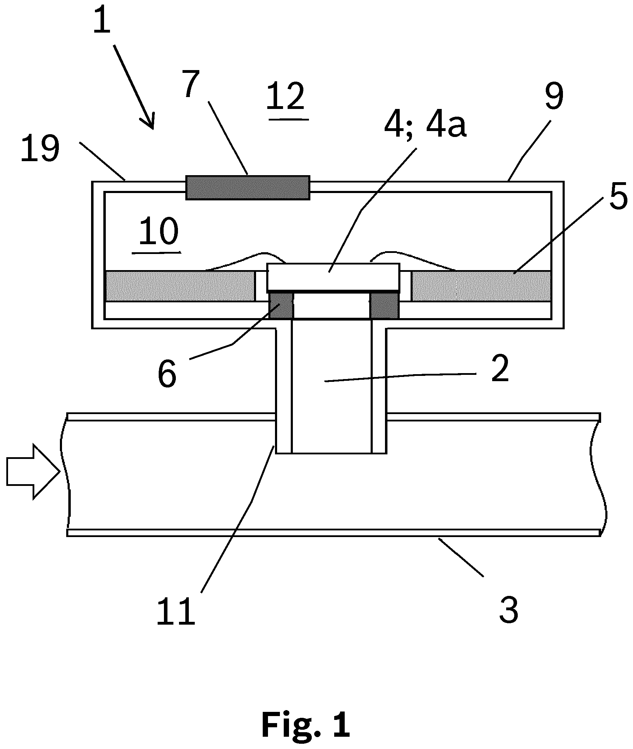

- Fig. 1: zeigt einen Strömungskanal eines Wasserstoff-Systems mit einem Ausführungsbeispiel eines angeschlossenen elektrischen Geräts, dessen Betriebskomponente als Sensorelement ausgebildet ist.

### Ausführungsformen der Erfindung

Fig. 1 zeigt ein Ausführungsbeispiel eines elektrischen Gerätes 1, welches beispielhaft als Sensor ausgebildet ist. Das elektrische Gerät 1 weist ein Gehäuse 9 aus beispielsweise thermoplastischem Material auf, welches einen Gehäuseinnenraum 10 birgt. Das Gehäuse 9 ist mit einem Stutzen 11 versehen, in dem ein Eintrittskanal 2 ausgebildet ist. Mittels des Stutzens 11 ist das elektrische Gerät 1 an einen Strömungskanal 3 eines Wasserstoff-Systems anschließbar. In dem Strömungskanal 3 in Fig. 1 strömt ein Betriebsgas in der dargestellten Pfeilrichtung. Das Betriebsgas enthält einen Wasserstoffanteil. Das in dem Strömungskanal 3 strömende Betriebsgas gelangt über den Eintrittskanal 2 zum Gehäuse 9. Das Gehäuse 9 weist einen Gehäuseinnenraum 10 auf, in dem eine Betriebskomponente 4 angeordnet, bei der es sich beispielsweise um ein Sensorelement 4a handelt. Das Sensorelement 4a kann über beispielsweise als Bonddrahtverbindungen ausgebildete elektrische Verbindungen mit einem in dem Gehäuseinnenraum 10 angeordneten elektronischen Schaltungsteil 5 verbunden sein, auf dem eine Ansteuer- und Auswerteschaltung angeordnet ist. Das elektronische Schaltungsteil 5 kann über einen nicht dargestellten Gehäusestecker an einen elektrischen Gegenanschluss anschließbar sein. Das Sensorelement 4a weist eine dem Eintrittskanal 2 zu weisende Seite und eine gegenüberliegende dem Gehäuseinnenraum 10 zuweisende Seite auf. Vorzugsweise handelt es ich bei dem Sensorelement 4a um einen Wasserstoffkonzentrationssensor, der die Wasserstoffkonzentration in dem Eintrittskanal 2 ermittelt. Das Betriebsgas in dem Eintrittskanal 2 beaufschlagt das Sensorelement 4a auf seiner dem Eintrittskanal 2 zugewandten Seite. Das Sensorelement 4a ist beispielsweise wie in der DE 10 2014 212 430 A1 beschrieben als Messchip ausgebildet, und weist eine Membran auf, die mittels einer Heizleiterbahn gegenüber der Umgebung beheizbar ist. Dadurch entsteht ein Wärmefluss, welcher vom Mischungsverhältnis des Betriebsgases abhängt. Durch Auswertung der Temperaturen der Heizleistungen mittels der auf dem elektronischen Schaltungsteil 5 angeordneten Auswerteschaltung kann dann beispielsweise die Wasserstoffkonzentration oder die Feuchtigkeit gemessen werden. Aber auch anderer Ausgestaltungen sind denkbar.

Wie in Fig. 1 weiterhin erkennbar ist, dichtet ein erstes Dichtelement 6 den Bereich zwischen dem Eintrittskanal 2 und dem Sensorelement 4a ab. Das erste Dichtelement 6 kann beispielsweise ein Elastomer sein. Auch andere Materialien sind möglich. Es kann sich um einen Dichtring oder eine Dichtklebung handelt. Das erste Dichtelement 6 umgibt die Öffnung des Eintrittskanals 2 an der Gehäuseinnenseite umlaufend und liegt mit einer ersten Dichtfläche an der Gehäuseinnenseite der Gehäusewand 9 und mit einer zweiten Dichtfläche an dem Sensorelement 4a an. Dadurch wird der mit dem Betriebsgas gefüllte Bereich zwischen dem Eintrittskanal 2 und der mit dem Betriebsgas beaufschlagten Seite des Sensorelements 4a gegenüber dem Gehäuseinnenraum 10 abgedichtet. Wasserstoff kann jedoch durch Permeation von dem Eintrittskanal 2 aus durch das erste Dichtelement 5 hindurch in den Gehäuseinnenraum 10 gelangen. Daher weist die Gehäusewand 9 des Gehäuses 1 eine mit einem zweiten Dichtelement 7 verschlossene Öffnung auf. Das zweite Dichtelement 7 kann beispielsweise als Membran ausgebildet sein. Der in den Gehäuseinnenraum 10 eindringende Wasserstoff kann durch Permeation des zweiten Dichtelementes 7 vorteilhaft in den Außenraum 12 entweichen. Wichtig ist, dass die Permeation des Wasserstoffs durch das zweite Dichtelement 7 gleich oder größer ist als die Permeation des Wasserstoffs durch das erste Dichtelement 6 in den Gehäuseinnenraum 10. Ist diese Bedingung erfüllt, steigt die Wasserstoffkonzentration im Gehäuseinnenraum 10 nicht an.

Das zweite Dichtelement 7 kann beispielsweise aus dem gleichen Material oder einem sehr ähnlichen Material wie das erste Dichtelement 6 gefertigt sein, beispielsweise aus einem Elastomer. Das zweite Dichtelement 7 kann als Dichtkörper oder Dichtklebeverbindung ausgebildet sein. Die Permeabilität des zweiten Dichtelementes 7 kann durch die Flächenausdehnung des zweiten Dichtelementes 7 beziehungsweise dessen Querschnitt in der Öffnung der Gehäusewand 9 relativ leicht eingestellt werden. Je größer der Querschnitt des zweiten Dichtelementes 7 ist, desto größer ist die Permeation des Wasserstoffs in den Außenraum 12. Darüber hinaus besteht natürlich auch die Möglichkeit durch die Auswahl der Dicke oder die Materialauswahl die Permeation des Wasserstoffs durch das zweite Dichtelement 7 auf einen Wert einzustellen, der gleich oder größer ist als die Permeation des Wasserstoffs durch das erste Dichtelement 6.

Das vorliegende Ausführungsbeispiel wurde anhand eines Sensorelementes 4a erläutert. Es versteht sich jedoch, dass ein ähnlicher Aufbau unter Beibehaltung der erfindungswesentlichen Merkmale auch für eine Betriebskomponente 4 verwandt werden kann, die nicht als Sensor, sondern als Aktuator, insbesondere als elektrische betätigtes Ventil ausgestaltet ist.

## Patentansprüche

1. Elektrisches Gerät (1) für den Einsatz in einem System, welches mit einem Wasserstoff enthaltenden Betriebsgas betrieben wird, umfassend: ein Gehäuse (9) mit einem an dem Gehäuse (9) ausgebildeten Eintrittskanal (2) für das Betriebsgas und mit wenigstens einem Gehäuseinnenraum (10), in dem ein elektronisches Schaltungsteil (5) angeordnet ist, und mit einer mit dem elektronischen Schaltungsteil (5) elektrisch verbundenen, in dem Gehäuse (9) angeordneten Betriebskomponente (4), wobei der Eintrittskanal (2) über ein in dem Gehäuse (9) angeordnetes erstes Dichtelement (6) an die Betriebskomponente (4) angebunden ist und die Betriebskomponente (4) über den Eintrittskanal (2) mit Betriebsgas beaufschlagbar ist, wobei das erste Dichtelement (6) den Eintrittskanal (2) gegenüber dem Gehäuseinnenraum (10) bis auf eine verbleibende Wasserstoffpermeabilität abdichtet, **dadurch gekennzeichnet, dass** das Gehäuse weiterhin wenigstens ein den Gehäuseinnenraum (10) zum Außenraum (12) hin abdichtendes zweites Dichtelement (7) aufweist, wobei das zweite Dichtelement (7) eine Wasserstoffpermeabilität aufweist, die gleich oder größer als die Wasserstoffpermeabilität des ersten Dichtelementes (6) ist.

2. Elektrisches Gerät nach Anspruch 1, **dadurch gekennzeichnet, dass** das zweite Dichtelement (7) in eine Gehäusewand (19) des Gehäuses (9) eingelassen ist.

3. Elektrische Gerät nach Anspruch 1 oder 2, **dadurch gekennzeichnet, dass** das zweite Dichtelement (7) als Membran ausgebildet ist.

4. Elektrisches Gerät nach einem der vorstehenden Ansprüchen, **dadurch gekennzeichnet, dass** das zweite Dichtelement (7) aus dem gleichen oder einem anderen Material als das erste Dichtelement (6) besteht.

5. Elektrisches Gerät nach einem der vorstehenden Ansprüche, **dadurch gekennzeichnet, dass** das erste Dichtelement (6) und/oder das zweite Dichtelement (7) aus einem Elastomer-Dichtkörper oder einem Dichtklebstoff bestehen.

6. Elektrisches Gerät nach einem der vorstehenden Ansprüche, **dadurch gekennzeichnet, dass** das elektrische Gerät (1) mit einem den Eintrittskanal (2) umgebenden Stutzen (11) an einen das Betriebsgas führenden Strömungskanal (3) anschließbar ist, insbesondere an einen Anodenpfad eines Brennstoffzellensystems.

7. Elektrisches Gerät nach einem der vorstehenden Ansprüche, **dadurch gekennzeichnet, dass** die Betriebskomponente (4) als ein Sensorelement (4a) zur Erfassung mindestens einer Eigenschaft des Betriebsgases ausgebildet ist.

8. Elektrisches Gerät nach Anspruch 7, **dadurch gekennzeichnet, dass** das Sensorelement (4a) ausgewählt ist auf der folgenden Gruppe von Sensorelementen:
- ein Sensorelement zur Erfassung der Wasserstoffkonzentration von Wasserstoff in dem Betriebsgas,
- ein Sensorelement zur Erfassung des Drucks des Betriebsgases,
- ein Sensorelement zur Erfassung der Temperatur des Betriebsgases,
- ein Sensorelement zur Erfassung der Feuchtigkeit in dem Betriebsgas.

9. Elektrisches Gerät nach einem der Ansprüche 1 bis 6, **dadurch gekennzeichnet, dass** die Betriebskomponente (4) ein elektrisch betriebener Aktuator, insbesondere ein elektrisch betriebenes Dosierventil ist.

10. Wasserstoff-System mit einem elektrischen Gerät (1) nach einem der Ansprüche 1 bis 9.

## Claims

1. Electrical apparatus (1) for use in a system which is operated with an operating gas containing hydrogen, comprising: a housing (9) with an inlet channel (2) for the operating gas, formed on the housing (9), and with at least one housing interior (10), in which an electronic circuit component (5) is arranged, and with an operating component (4), which is electrically connected to the electronic circuit component (5) and is arranged in the housing (9), wherein the inlet channel (2) is attached to the operating component (4) by way of a first sealing element (6) arranged in the housing (9) and operating gas can be applied to the operating component (4) by way of the inlet channel (2), wherein the first sealing element (6) seals off the inlet channel (2) from the housing interior (10) apart from a remaining hydrogen permeability, **characterized in that** the housing also has at least one second sealing element (7), sealing off the housing interior (10) with respect to the outside (12), wherein the second sealing element (7) has a hydrogen permeability which is equal to or greater than the hydrogen permeability of the first sealing element (6).

2. Electrical apparatus according to Claim 1, **characterized in that** the second sealing element (7) is let into a housing wall (19) of the housing (9).

3. Electrical apparatus according to Claim 1 or 2, **characterized in that** the second sealing element (7) takes the form of a membrane.

4. Electrical apparatus according to one of the preceding claims, **characterized in that** the second sealing element (7) consists of the same material as the first sealing element (6) or a different material.

5. Electrical apparatus according to one of the preceding claims, **characterized in that** the first sealing element (6) and/or the second sealing element (7) consist(s) of an elastomer sealing body or a sealing adhesive.

6. Electrical apparatus according to one of the preceding claims, **characterized in that** the electrical apparatus (1) can be connected by a connecting piece (11) surrounding the inlet channel (2) to a flow channel (3) carrying the operating gas, in particular to an anode path of a fuel cell system.

7. Electrical apparatus according to one of the preceding claims, **characterized in that** the operating component (4) takes the form of a sensor element (4a) for sensing at least one property of the operating gas.

8. Electrical apparatus according to Claim 7, **characterized in that** the sensor element (4a) is selected from the following group of sensor elements:
- a sensor element for sensing the hydrogen concentration of hydrogen in the operating gas,
- a sensor element for sensing the pressure of the operating gas,
- a sensor element for sensing the temperature of the operating gas,
- a sensor element for sensing the moisture in the operating gas.

9. Electrical apparatus according to one of Claims 1 to 6, **characterized in that** the operating component (4) is an electrically operated actuator, in particular an electrically operated metering valve.

10. Hydrogen system with an electrical apparatus (1) according to one of Claims 1 to 9.

## Revendications

1. Appareil électrique (1) destiné à être utilisé dans un système fonctionnant à l'aide d'un gaz de service contenant de l'hydrogène, comprenant un boîtier (9) pourvu d'un canal d'entrée (2) formé sur le boîtier (9) pour le gaz de service et comprenant au moins un espace intérieur (10) dans lequel est disposé un circuit électronique (5), et comprenant un composant fonctionnel (4) disposé dans le boîtier (9) et connecté électriquement au circuit électronique (5), le canal d'entrée (2) étant relié au composant fonctionnel (4) par l'intermédiaire d'un premier élément d'étanchéité (6) disposé dans le boîtier (9) et le composant fonctionnel (4) pouvant être alimenté en gaz de service par l'intermédiaire du canal d'entrée (2), le premier élément d'étanchéité (6) assurant l'étanchéité du canal d'entrée (2) par rapport à l'espace intérieur du boîtier (10), à l'exception d'une perméabilité résiduelle à l'hydrogène, **caractérisé en ce que** le boîtier comporte en outre au moins un deuxième élément d'étanchéité (7) assurant l'étanchéité de l'espace intérieur du boîtier (10) par rapport à l'espace extérieur (12), le deuxième élément d'étanchéité (7) présentant une perméabilité à l'hydrogène égale ou supérieure à la perméabilité à l'hydrogène du premier élément d'étanchéité (6).

2. Appareil électrique selon la revendication 1, **caractérisé en ce que** le deuxième élément d'étanchéité (7) est incorporé dans une paroi de boîtier (19) du boîtier (9).

3. Appareil électrique selon la revendication 1 ou 2, **caractérisé en ce que** le deuxième élément d'étanchéité (7) est réalisé sous forme de membrane.

4. Appareil électrique selon l'une des revendications précédentes, **caractérisé en ce que** le deuxième élément d'étanchéité (7) est constitué du même matériau ou d'un matériau différent de celui du premier élément d'étanchéité (6).

5. Appareil électrique selon l'une des revendications précédentes, **caractérisé en ce que** le premier élément d'étanchéité (6) et/ou le deuxième élément d'étanchéité (7) est/sont constitué(s) d'un corps d'étanchéité en élastomère ou d'une colle d'étanchéité.

6. Appareil électrique selon l'une des revendications précédentes, **caractérisé en ce que** l'appareil électrique (1) peut être raccordé, par l'intermédiaire d'un embout (11) entourant le canal d'entrée (2), à un canal d'écoulement (3) transportant le gaz de service, en particulier à un chemin d'anode d'un système de pile à combustible.

7. Appareil électrique selon l'une des revendications précédentes, **caractérisé en ce que** le composant fonctionnel (4) est réalisé sous la forme d'un élément capteur (4a) destiné à détecter au moins une propriété du gaz de service.

8. Appareil électrique selon la revendication 7, **caractérisé en ce que** l'élément capteur (4a) est sélectionné dans le groupe d'éléments de capteur suivant :
- un élément capteur pour détecter la concentration en hydrogène du gaz fonctionnel,
- un élément capteur pour détecter la pression du gaz de service,
- un élément capteur pour détecter la température du gaz de service,
- un élément capteur pour détecter l'humidité dans le gaz de service.

9. Appareil électrique selon l'une des revendications 1 à 6, **caractérisé en ce que** le composant fonctionnel (4) est un actionneur électrique, en particulier une vanne de dosage électrique.

10. Système à hydrogène comprenant un appareil électrique (1) selon l'une des revendications 1 à 9.
